# EUROPEAN PATENT SPECIFICATION

(11) **EP 1 159 620 B1**
(45) Date of publication and mention of the grant of the patent: **11.05.2005**
(21) Application number: 00907799.1
(22) Date of filing: 03.03.2000
(51) Int. Cl.: G01N 33/68, A61K 45/00, C07K 7/00

(54) **DETECTION OF MTOC DISRUPTING SUBSTANCES**
NACHWEIS MTOC VERRENKENDEN SUBSTANZEN
DETECTION DE SUBSTANCES DE RUPTURE D'INTEGRITE DE CENTRE ORGANISANT LES MICROTUBULES

(30) Priority: 04.03.1999 GB 9905007
(43) Date of publication of application: 05.12.2001
(73) Proprietor: Cyclacel Limited, London SW1Y 4RB (GB)
(72) Inventor: GLOVER, David, Moore, Sandy, Bedfordshire SG19 3AA (GB); AVIDES, Maria do Carmo, Cambridge CB3 9HD (GB)
(74) Representative: Nachshen, Neil Jacob
(86) International application number: PCT/GB2000/000785
(87) International publication number: WO 2000/052478

(56) References cited:
- WO-A-97/06440
- MEDLINE, Washington DC USA; abstract no. 97296495, whole abstract XP002140154 cited in the application & R.D. SAUNDERS ET AL.: "The Drosophila gene abnormal spindle encodes a novel microtubule-associated protein that associates with the polar regions of the mitotic spindle " JOURNAL OF CELL BIOLOGY, vol. 137, no. 4, 19 May 1997 (1997-05-19), pages 881-890, New York NY USA
- MEDLINE, Washington DC USA; abstract no. 98120401, abstract lines 14-17 XP002140155 & A BANAN ET AL.: "Protection against ethanol injury by prostaglandin in a human intestinal cell line: role of microtubules " AMERICAN JOURNAL OF PHYSIOLOGY, vol. 274, no. 1 PT 1, 1 January 1998 (1998-01-01), pages G111-G121, St. Loius MI USA

## Description

### Field of the Invention

The present invention relates to the use of the Asp protein in a method of identifying substances capable of arresting cells in mitosis by disrupting microtubule organising centre formation and/or maintenance. Such substances may be used in therapeutic methods to inhibit cell growth.

### Background to the Invention

The aim of many anti-cancer therapies is to inhibit cell division, i.e. mitosis, in tumour cells. Assembly of the spindle apparatus is a key event in cell mitosis since the spindle apparatus is required for chromosome segregation between daughter cells. Spindle apparatus assembly is thus a potential target for inhibition of mitosis and tumour therapy.

The spindle apparatus comprises microtubules which are organised at its poles by a microtubule organising centre (MTOC) that in most animal cells is termed the centrosome. The centrosome itself usually comprises a pair of centrioles associated with a cloud of poorly defined pericentriolar material (PCM). During interphase, the PCM nucleates a cytoplasmic array of microtubules that project outward toward the cell perimeter. Duplication of the centrosome occurs during interphase. The two centrosomes remain together as a single complex prior to mitosis but as mitosis begins, this complex splits in two. Each centrosome then serves as a separate MTOC that nucleates a radial array of microtubules called an aster. The two asters move to opposite sides of the nucleus to form the two poles of the mitotic spindle.

Several mutations have been identified in a variety of experimental organisms that result in defects in spindle formation. Once such mutation, the *asp* mutation, was originally identified in *Drosophila* by Ripoll *et al*., 1975. The *asp* gene has been cloned in *Drosophila* and shown to encode a highly basic protein with a molecular weight of 220 kDa comprising putative actin and calmodulin binding domains (Saunders *et al*., 1997).

The abnormal spindle microtubules seen in meiosis and in mitosis of a variety of cell types led Saunders *et al*., 1997 to speculate that the *asp* gene product was involved in some aspect of spindle microtubule dynamics. The association of Asp with microtubules was demonstrated by the finding that it copurified with taxol stabilised microtubules and remained associated following a wash with 0.4 M NaCl. Immunolocalisation of the Asp protein within syncytial embryos was consistent with this finding. The Asp protein was present in the cytoplasm during interphase, and on microtubules in the polar regions of the spindle in mitosis until telophase, when it became associated with the central region of the spindle (Saunders *et al*., 1997).

### Summary of the Invention

We have now shown that, contrary to previous findings indicating that Asp is a microtubule associated protein localised to the centrosomes of mitiotic spindles, Asp is in fact associated with the centrosome throughout mitosis and is present on the centrosome following its isolation *in vitro*. Furthermore, we have shown that depletion of Asp from *Drosophila* embryo extracts results in the loss of the ability of the extracts to restore microtubule organising centre (MTOC) activity to salt-stripped centrosome preparations. We have also shown that antibodies directed against the Asp protein block microtubule nucleation from the centrosomes in a dose-dependent manner.

Thus substances that bind to Asp and interfere with the interaction of Asp with the centrosome may be used to disrupt MTOC integrity and consequently normal spindle formation, resulting in mitotic arrest. These substances may therefore also be used to inhibit mitosis and cellular proliferation.

Accordingly the present invention provides a method for identifying a substance capable of disrupting microtubule organising centre (MTOC) integrity, which method comprises contacting an Asp polypeptide as shown in SEQ ID NO: 1 or fragment thereof capable of forming and/or maintaining MTOCs in the absence of the substance, with a candidate substance in the presence of components required for MTOC formation and microtubule nucleation therefrom, and determining whether the substance disrupts MTOC integrity.

The term "disrupting MTOC integrity" means reducing or preventing the formation and/or maintenance of MTOCs, particularly centrosomes, capable of nucleating microtubules assembly from an organised centre to produce aster-like polymerised microtubule structures, such as asters themselves.

In one preferred embodiment, said components comprise KI-extracted centrosomes and an Asp-depleted soluble cellular extract. In another preferred embodiment, said components comprise a partially purified centrosome preparation and tubulin.

Candidate substances identified by the assay method of the invention may be further tested in mitosis inhibition assays comprising administering substance, which has been determined to disrupt MTOC integrity by the assay method described above, to a cell and determining whether the substance inhibits mitosis in the cell.

The present invention also provides the use of an Asp polypeptide as shown in SEQ ID NO: 1 or fragment thereof capable of stimulating formation of and/or maintaining MTOCs, in an assay for identifying a substance capable of disrupting MTOC integrity.

### Detailed Description of the Invention

Although in general the techniques mentioned herein are well known in the art, reference may be made in particular to Sambrook *et al*., Molecular Cloning, A Laboratory Manual (1989); Ausubel *et al*., Current Protocols in Molecular Biology (1995) and John Wiley & Sons, Inc.

### A. Asp polypeptides

Asp polypeptides for use in the assay methods of the present invention include the *Drosophila* Asp polypeptide, described in Saunders *et al*., 1997, J. Cell Biol., Vol. 137, 4, p.881-890, and homologues, variants, derivatives and fragments thereof. The amino acid and nucleotide sequence of *Drosophila* Asp described in Saunders *et al*., 1997 is shown as SEQ I.D. No 1.

With respect to fragments, truncated versions of the above may be used in the assays of the present invention provided that the fragments are capable of stimulating MTOC formation and/or maintenance as determined by the ability of the MTOCs to nucleate asters.

The term "microtubule nucleation centre" means a centrosome that is capable of acting as a centre for microtubule nucleation. Preferably, the centrosome is capable of acting as a centre for microtubule nucleation during mitosis. Thus the term "microtubule nucleation centre" refers to an intact MTOC (centrosome) that is capable of nucleating asters of microtubules.

The term "microtubule organising centre integrity" used throughout includes both the formation of MTOCs and their maintenance in a state capable of nucleating asters.

Asp polypeptides for use in the invention may be made by recombinant means, for example as described below. However they may also be made by synthetic means using techniques well known to skilled persons such as solid phase synthesis. Polypeptides for use in the invention may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein of interest in maintaining microtubule organising centre integrity.

Alternatively, Asp polypeptides may be purified from vertebrate cells, such as insect or mammalian cells. A detailed protocol is described in section E.

Proteins for use in the invention may be in a substantially isolated form. It will be understood that the protein may be mixed with carriers or diluents which will not interfere with the intended purpose of the protein and still be regarded as substantially isolated. A protein of the invention may also be in a substantially purified form, in which case it will generally comprise the protein in a preparation in which more than 90%, e.g. 95%, 98% or 99% of the protein in the preparation is an Asp polypeptide.

### B. Polynucleotides

Polynucleotides for use according to the invention comprise nucleic acid sequences encoding the amino acid sequences of the invention. They also include the *Drosophila* Asp cDNA sequence shown as SEQ I.D. No. 2, and fragments. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides for use according to the invention to reflect the codon usage of any particular host organism in which the polypeptides of the invention are to be expressed.

Polynucleotides for use according to the present invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides of the invention.

Polynucleotides for use according to the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides for use in the invention as used herein.

Polynucleotides such as a DNA polynucleotides and probes for use according to the invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the Asp sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

### C. Nucleotide vectors

Polynucleotides for use in the present invention can be incorporated into a recombinant replicable vector. The vector may be used to replicate the nucleic acid in a compatible host cell. Thus in a further embodiment, the invention provides a method of making polynucleotides for use in the invention by introducing a polynucleotide of the invention into a replicable vector, introducing the vector into a compatible host cell, and growing the host cell under conditions which bring about replication of the vector. The vector may be recovered from the host cell. Suitable host cells include bacteria such as *E. coli*, yeast, mammalian cell lines and other eukaryotic cell lines, for example insect Sf9 cells.

Preferably, a polynucleotide for use according to the invention in a vector is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

Vectors for use according to the invention may be transformed or transfected into a suitable host cell as described below to provide for expression of a protein of the invention. This process may comprise culturing a host cell transformed with an expression vector as described above under conditions to provide for expression by the vector of a coding sequence encoding the protein, and optionally recovering the expressed protein.

The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used, for example, to transfect or transform a host cell.

Control sequences operably linked to sequences encoding Asp polypeptides include promoters/enhancers and other expression regulation signals. These control sequences may be selected to be compatible with the host cell for which the expression vector is designed to be used in. The term promoter is well-known in the art and encompasses nucleic acid regions ranging in size and complexity from minimal promoters to promoters including upstream elements and enhancers.

The promoter is typically selected from promoters which are functional in mammalian cells, although prokaryotic promoters and promoters functional in other eukaryotic cells, in particular insect cells, may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

### D. Host cells

Vectors and polynucleotides for use according to the invention may be introduced into host cells for the purpose of replicating the vectors/polynucleotides and/or expressing Asp polypeptides. Although the Asp polypeptides may be produced using prokaryotic cells as host cells, it is preferred to use eukaryotic cells, for example yeast, insect or mammalian cells, in particular mammalian cells.

Vectors/polynucleotides for use in the invention may introduced into suitable host cells using a variety of techniques known in the art, such as transfection, transformation and electroporation. Where vectors/polynucleotides of the invention are to be administered to animals, several techniques are known in the art, for example infection with recombinant viral vectors such as retroviruses, herpes simplex viruses and adenoviruses, direct injection of nucleic acids and biolistic transformation.

### E. Protein Expression and Purification

Host cells comprising polynucleotides for use according to the invention may be used to express proteins for use according to the invention. Host cells may be cultured under suitable conditions which allow expression of the Asp polypeptides. Expression of the Asp polypeptides may be constitutive such that they are continually produced, or inducible, requiring a stimulus to initiate expression. In the case of inducible expression, protein production can be initiated when required by, for example, addition of an inducer substance to the culture medium, for example dexamethasone or IPTG.

Proteins for use according to the invention can be extracted from host cells by a variety of techniques known in the art, including enzymatic, chemical and/or osmotic lysis and physical disruption.

Polypeptides for use in the present invention may also be purified from extracts of cells in which they naturally occur. For example, *Drosophila* Asp may be purified from *Drosophila* embryos. Source cells may be, for example, obtained from whole organisms or from cells in tissue culture. A particular purification protocol for *Drosophila* Asp from embryos is as follows.

Drosophila embryos are collected over a three hour period and dechorionated by incubation in 50% sodium hypochloride for 3 min. The bleach is then removed by extensive washes with water. Embryos are then washed in lysis buffer (0.1 M Pipes/NaOH, pH 6.6, 5 mM EGTA, 1 mM MgSO₄, 0.9 M glycerol, 1 mM DTT, 1 mM PMSF, 1 mg/ml aprotinin, 1 mg/ml leupeptin and 1 mg/ml pepstatin). About 3 ml of embryos are homogenised in 2 volumes of ice-cold lysis buffer with a Dounce homogeniser or a pestle and mortar. The microtubules are depolymerised by incubation on ice for 15 min and the extract centrifuged at 16,000 g for 30 min at 4°C. The supernatant is recentrifuged at 135,000 g for 90 min at 4°C. Microtubules in the resulting supernatant are polymerised by addition of GTP to a final concentration of 1 mM, addition of taxol to a final concentration of 20 µM and incubation at room temperature for 30 min. 3 ml aliquots of extract are layered on top of 3 ml 15% sucrose cushions prepared in lysis buffer supplemented with 20 µM taxol and 1 mM GTP. After centrifuging at 54,000 g for 30 min at 20°C using a swing out rotor, the pellet is suspended in lysis buffer containing taxol and GTP. This also provides a suitable cell extract for use in microtubule nucleation assays described below.

To extract the microtubule associated proteins, the pellet is extracted with 400-500 mM NaCl. After centrifugation, the pellet is them extracted with 1 M NaCl, centrifuged and finally extracted with 1-2 M KI. Purified Asp protein is present in the soluble KI fraction which is then typically concentrated ultrafiltration using Millipore Ultrafree systems. Fractions may also be dialysed against a suitable buffer prior to storage and use.

We have shown that using this purification technique, the protein retains biological activity and when added to an asp-mutant embryo extract it can correct the defect in organisation of the microtubule nucleating activity of centrosomes. Modified protocols will be applicable to other types of organisms. For example, protocols are known to the skilled person for the purification of mammalian microtubules and their associated proteins (MAPs). Once the MAPs have been extracted according to these protocols using NaCl, the Asp protein may typically still be present in the pellet. The Asp protein may then be extracted with 1-2 M KI. Exact conditions may vary but may easily be determined by a person skilled in fractionation of cellular extracts.

### F. Assays

The assays of the present invention are suitable for identifying substances that inhibit microtubule nucleation centre organising activity mediated by an Asp polypeptide (reference to which includes homologues, variants, derivatives and fragments as described above). Such assays are typically *in vitro.* Assays are also provided that test the effects of candidate substances identified in preliminary *in vitro* assays on intact cells in whole cell assays.

### Candidate substances

A substance that inhibits microtubule nucleation centre organising activity (i.e. maintenance of MTOC integrity) mediated by an Asp polypeptide may do so in several ways. It may directly disrupt the binding Asp to a component of the centrosome of the two components by, for example, binding to Asp and masking or altering the site of interaction with the other component. Candidate substances of this type may conveniently be preliminarily screened by *in vitro* binding assays as, for example, described below and then tested in a microtubule nucleation assay as described below. Examples of candidate substances include antibodies which recognise Asp.

A substance which can bind directly to Asp may also inhibit its microtubule nucleation centre organising activity by altering its subcellular localisation thus preventing Asp and components of the centrosome from coming into contact within the cell. This can be tested using, for example the whole cells assays described below. Non-functional homologues of Asp may also be tested for inhibition of microtubule nucleation centre organising activity since they may compete with Asp for binding to components of the centrosome whilst being incapable of stimulating microtubule organising centre nucleation activity or block the function of Asp bound to the centrosome. Such non-functional homologues may include naturally occuring Asp mutants and modified Asp sequences or fragments thereof.

Alternatively, instead of preventing the association of the components directly, the substance may suppress the biologically available amount of Asp. This may be by inhibiting expression of the component, for example at the level of transcription, transcript stability, translation or post-translational stability. An example of such a substance would be antisense RNA or double-stranded interfering RNA sequences which suppresses the amount of Asp mRNA biosynthesis.

Suitable candidate substances include peptides, especially of from about 5 to 30 or 10 to 25 amino acids in size, based on the sequence of the various domains of Drosophila *Asp* described in section A, or variants of such peptides in which one or more residues have been substituted. Peptides from panels of peptides comprising random sequences or sequences which have been varied consistently to provide a maximally diverse panel of peptides may be used.

Particularly preferred peptides include peptides containing, for example, one or more p34^{cdc2} consensus phosphorylation sites (such as residues 267-271, 272-276, 291-295, 387-391, 421-425 or 473-477 of SEQ I.D. No. 1), one or more MAP kinase consensus phosphorylation sites (such as residues 140-144, 237-241, 269-273 or 471-475 of SEQ I.D. No. 1), one or more monoclonal antibody MPM2 epitope phosphorylation sites (such as residues 155-160, or 237-243 of SEQ I.D. No. 1), a putative actin binding site (such as residues 743-875 of SEQ I.D. No. 1) or one or more IQ motifs (sites of interaction with members of the calmodulin family of proteins (Cheny and Mooseker, 1992)) (such as residues 911-940, 1293-1322, 1374-1403, 1563-1591 or 1597-1624 of SEQ I.D. No. 1). These peptides may be modified by insertion, deletion or substitution (including the use of non-naturally occuring amino acids and analogues).

Thus preferred peptides include peptides consisting essentially of from 5 to 35 amino acids comprising a amino acid sequence selected from residues x-y of SEQ I.D. No. 1 wherein x-y is 267-271, 272-276, 291-295, 387-391, 421-425, 473-477, 140-144, 237-241, 269-273, 471-475, 155-160, 237-243, 743-875, 911-940, 1293-1322, 1374-1403, 1563-1591 and 1597-1624.

Suitable candidate substances also include antibody products (for example, monoclonal and polyclonal antibodies, single chain antibodies, chimeric antibodies and CDR-grafted antibodies) which are specific for Asp. Furthermore, combinatorial libraries, peptide and peptide mimetics, defined chemical entities, oligonucleotides, and natural product libraries may be screened for activity as inhibitors of Asp-mediated microtubule nucleation centre organising activity in assays such as those described below. The candidate substances may be used in an initial screen in batches of, for example 10 substances per reaction, and the substances of those batches which show inhibition tested individually. Candidate substances which show activity in *in vitro* screens such as those described below can then be tested in whole cell systems, such as mammalian cells which will be exposed to the inhibitor and tested for inhibition of mitosis.

### Asp binding assays

One type of preliminary assay for identifying substances that bind to Asp involves contacting an Asp polypeptide, which is immobilised on a solid support, with a non-immobilised candidate substance determining whether and/or to what extent the Asp polypeptide and candidate substance bind to each other. Alternatively, the candidate substance may be immobilised and the Asp polypeptide non-immobilised.

In a preferred assay method, the Asp polypeptide is immobilised on beads such as agarose beads. Typically this is achieved by expressing the component as a GST-fusion protein in bacteria, yeast or higher eukaryotic cell lines and purifying the GST-fusion protein from crude cell extracts using glutathione-agarose beads (Smith and Johnson, 1988). As a control, binding of the candidate substance, which is not a GST-fusion protein, to the immobilised Asp polypeptide is determined in the absence of the Asp polypeptide. The binding of the candidate substance to the immobilised Asp polypeptide is then determined. This type of assay is known in the art as a GST pulldown assay. Again, the candidate substance may be immobilised and the Asp polypeptide non-immobilised.

It is also possible to perform this type of assay using different affinity purification systems for immobilising one of the components, for example Ni-NTA agarose and histidine-tagged components.

Binding of the Asp polypeptide to the candidate substance may be determined by a variety of methods well-known in the art. For example, the non-immobilised component may be labelled (with for example, a radioactive label, an epitope tag or an enzyme-antibody conjugate). Alternatively, binding may be determined by immunological detection techniques. For example, the reaction mixture can be Western blotted and the blot probed with an antibody that detects the non-immobilised component. ELISA techniques may also be used.

### Microtubule organising centre nucleation activity assays

Candidate substances, for example those identified using the Asp binding assays described above, may be screening using a microtubule organising centre nucleation activity assay to determine if they are capable of disrupting MTOCs as measured by, for example, aster formation. This assay in its simplest form comprises adding the candidate substance to a cellular extract which in the absence of the candidate substance has microtubule organising centre nucleation activity resulting in formation of asters.

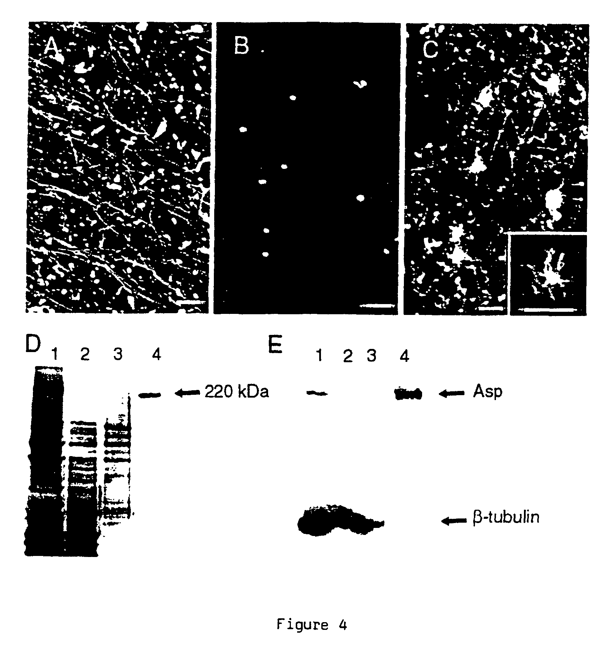

In a preferred embodiment, the assay system comprises (i) an Asp polypeptide or homologue, variant, derivative, fragment thereof and (ii) components required for microtubule organising centre nucleation activity except for functional Asp, which is typically removed by immunodepletion (or by the use of extracts from Asp mutants). The components themselves are typically in two parts such that microtubule nucleation does not occur until the two parts are mixed (microtubule nucleation can take place in the absence of Asp but the result is a mass of disorganised microtubules rather than normal aster - see Figure 4A vs Figure 4C). The Asp may be present in one of the two parts intially or added subsequently prior to mixing of the two parts.

Subsequently, the Asp polypeptide and candidate substance are added to the component mix and microtubule nucleation from centrosomes measured, for example by immunostaining for Asp and visualising aster formation by immuno-fluorescence microscopy. Asp polypeptide may be preincubated with the candidate substance before addition to the component mix. Alternatively, both Asp polypeptide and the candidate substance may be added directly to the component mix, simultaneously or sequentially in either order.

The components required for microtubule organising centre formation typically include salt-stripped centrosomes prepared as described in Moritz *et al*., 1998. Stripping centrosome preparations with 2 M KI removes the centrosome proteins CP60, CP190, CNN and γ-tubulin. Of these, neither CP60 nor CP190 appear to be required for microtubule nucleation. The other minimal components are typically provided as an Asp-depleted cellular extract, or conveniently, as a cellular extract from cells with non-functional Asp, for example *Drosophila* embryo extracts from asp-mutant embryos. Typically, labelled tubulin (usually β-tubulin) is also added to assist in visualising aster formation.

Alternatively, partially purified centrosomes that have not been salt-stripped may be used as part of the components. In this case, only tubulin, preferably labelled tubulin is required to complete the component mix.

Candidate substances are typically added to a final concentration of from 1 to 1000 nmol/ml, more preferably from 1 to 100 nmol/ml. In the case of antibodies, the final concentration used is typically from 100 to 500 µg/ml, more preferably from 200 to 300 µg/ml.

The degree of inhibition of aster formation by the candidate substance may be determined by measuring the number of normal asters per unit area for control untreated cell preperation and measuring the number of normal asters per unit area for cells treated with the candidate substance and comparing the result. Typically, a candidate substance is considered to be capable of disrupting MTOC integrity if the treated cell preparations have less than 50%, preferably less than 40, 30, 20 or 10% of the number of asters found in untreated cells preparations. It may also be desirable to stain cells for γ-tubulin to determine the maximum number of possible MTOCs present to allow normalisation between samples.

### Whole cell assays

Candidate substances may also be tested on whole cells for their affect microtubule organising centre integrity and downstream affects on mitosis. Preferably the candidate substances have been identified by the above-described *in vitro* methods. Alternatively, rapid throughout screens for substances capable of inhibiting mitosis may be used as a preliminary screen and then used in the *in vitro* assay described above to confirm that the affect is on MTOC intregrity.

The candidate substance, i.e. the test compound, may be administered to the cell in several ways. For example, it may be added directly to the cell culture medium or injected into the cell. Alternatively, in the case of polypeptide candidate substances, the cell may be transfected with a nucleic acid construct which directs expression of the polypeptide in the cell. Preferably, the expression of the polypeptide is under the control of a regulatable promoter.

Typically, an assay to determine the effect of a candidate substance identified by the method of the invention on cell mitosis comprises administering the candidate substance to a cell and determining whether the substance inhibits mitosis. Techniques for measuring mitosis in a cell population are well known in the art. The extent of mitosis in treated cells is compared with the extent of mitosis in an untreated control cell population to determine the degree of inhibition, if any. Cells may also be examined by fluorescence microscopy using suitable antibodies to microtubule/aster components to determine any effects on microtubule nucleation in treated cells.

The concentration of candidate substances used will typically be such that the final concentration in the cells is similar to that described above for the *in vitro* assays.

A candidate substance is typically considered to be an inhibitor of mitosis if mitosis is reduced to below 50%, preferably below 40, 30, 20 or 10% of that observed in untreated control cell populations.

### G. Therapeutic Uses

Since inhibition of microtubule nucleation will generally inhibit cellular mitosis, substances capable of inhibiting Asp-mediated maintenance or MTOCs, typically identified by the methods of the invention, may be used to inhibit mitosis, for example in rapidly dividing cells such as are found in tumour tissue. Thus substances capable of inhibiting Asp-mediated maintenance or MTOCs may be used in a method of inhibiting mitosis in a cell such as a mammalian cell, preferably a human cell. In a preferred embodiment, the cell is a tumour cell.

### H. Administration

Substances identified or identifiable by the assay methods of the invention may preferably be combined with various components to produce compositions of the invention. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline. The composition of the invention may be administered by direct injection. The composition may be formulated for parenteral, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to 1 mg/kg body weight.

Polynucleotides/vectors encoding polypeptide components for use in inhibiting mitosis may be administered directly as a naked nucleic acid construct, preferably further comprising flanking sequences homologous to the host cell genome. When the polynucleotides/vectors are administered as a naked nucleic acid, the amount of nucleic acid administered may typically be in the range of from 1 µg to 10 mg, preferably from 100 µg to 1 mg. It is particularly preferred to use polynucleotides/vectors that target specifically tumour cells, for example by virtue of suitable regulatory constructs or by the use of targeted viral vectors.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam™ and transfectam™). Typically, nucleic acid constructs are mixed with the transfection agent to produce a composition.

Preferably the polynucleotide or vector according to the invention is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular or transdermal administration.

The routes of administration and dosages described are intended only as a guide since a skilled practitioner will be able to determine readily the optimum route of administration and dosage for any particular patient and condition.

The invention will now be further described by way of Examples, which are meant to serve to assist one of ordinary skill in the art in carrying out the invention and are not intended in any way to limit the scope of the invention. The Examples refer to the Figures. In the Figures:

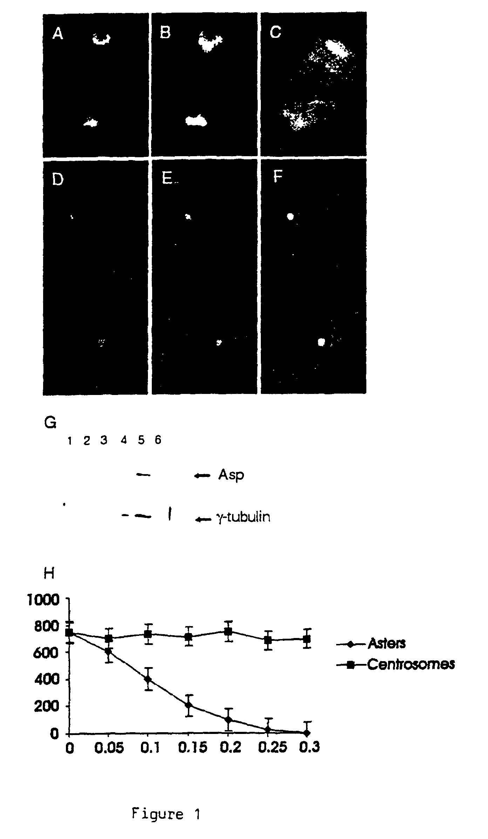

**Figure 1** Antibodies to Asp decorate the centrosome, and can block microtubule nucleating activity *in vitro.* [A through C] show the immunolocalisation of Asp in wild-type neuroblasts from *Drosophila* third instar larvae. A: Immunolocalisation of γ-tubulin (green), Asp (red), and DNA (blue) at metaphase. B: The same cell is shown as in A but only with Asp staining. C: An early anaphase cell showing α-tubulin(green), Asp (red), and DNA (blue). [D through F] show preparations of centrosomes from *Drosophila* embryos. D: γ-tubulin (blue). E: Asp (green). F: A merge of the two previous images also showing the asters of microtubules obtained after incubation with rhodamine-labelled tubulin. Panel G is a Immunoblot of the fractions from the final sucrose gradient centrifugation in the centrosome purification procedure. The 70% sucrose cushion was discarded, and the indicated fractions are the first six of a total of 26 in the remaining gradient. Asp and γ-tubulin co-sediment in fractions 4 and 5 as indicated. Fraction 5 was used in the experiments here. Panel H shows antibody competition assays in which centrosomes were first incubated with 0-0.3 µg/ml affinity purified anti-Asp, before being used in microtubule nucleation assays and finally being immunostained to reveal γ-tubulin. Preparations were scored for the total number of asters of microtubules and total centrosomes as indicated by foci of γ-tubulin . Scale bars, 10 µm.

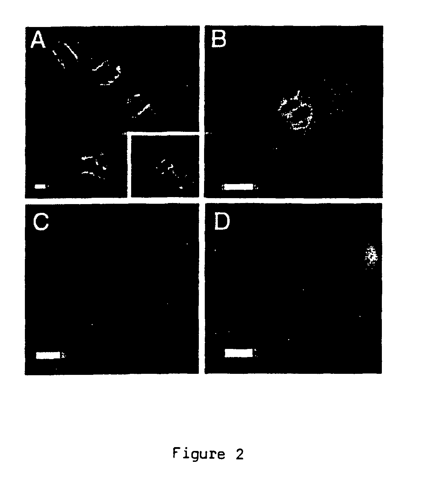

**Figure 2** Mitosis in *asp* mutants have unfocused spindle poles with disorganised γ-tubulin. Panels A and B show metaphase arrested cells in *asp*^{*dd4*} mutant brains in which α-tubulin is stained green and DNA is stained blue. A wild-type spindle is shown in the inset of panel A. Panels C and D show γ-tubulin (red) and DNA (blue) in *asp*^{*dd4*} brains (compare with the wild-type cell in Figure 1A). Scale bars, 10 µm.

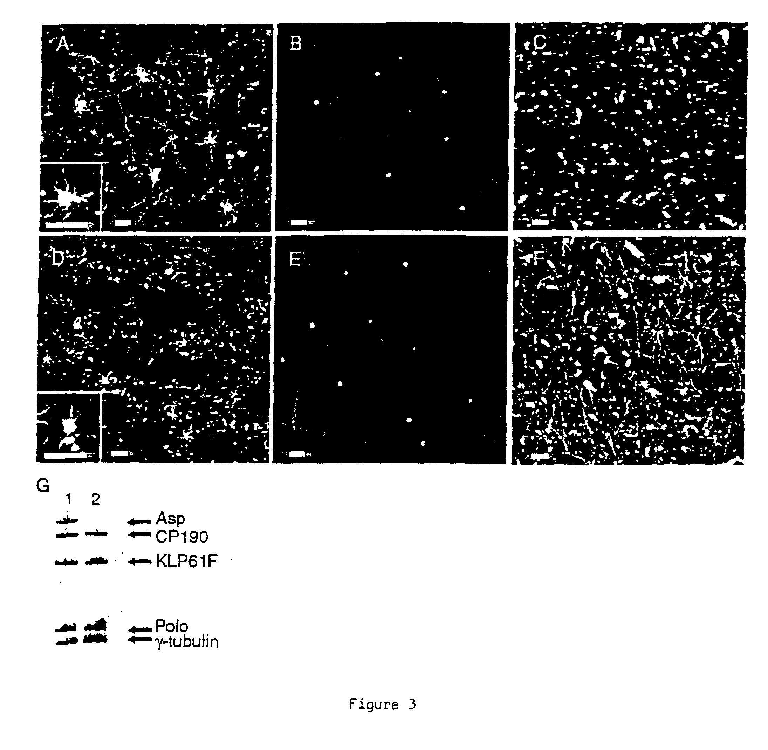

**Figure 3** Restoration of MTOC activity to salt stripped centrosomes by soluble embryo extracts is prevented by immunodepletion of Asp. A: Nucleation of rhodamine labelled microtubules from partially purified centrosomes. The inset shows a single centrosome at higher magnification. B: Same field as A immunostained to reveal γ-tubulin. Routinely, 80% of γ-tubulin staining bodies were seen to nucleate asters of microtubules. C: Microtubule nucleation after extraction of the centrosomes with 1 M KI. D: Potassium Iodide-extracted centrosomes incubated with soluble extract from wild-type *Drosophila* embryos before the microtubule nucleation assay. E: Same field as D immunostained to reveal γ-tubulin. F: Same field as D, but using an extract immunodepleted of Asp. Scale bars, 10 µm. G: Immunoblot of soluble embryo extract before (lane 1) and after immunodepletion of Asp (lane 2). The blot was probed with antibodies to Asp, CP190, KLP61F, polo and γ-tubulin as indicated.
**Figure 4** A soluble extract from *asp*-derived embryos failed to restore MTOC activity to salt stripped centrosomes, but this ability can be rescued by purified Asp protein. A and C show the nucleation of rhodamine-labelled microtubules by KI-extracted centrosomes incubated with a soluble extract from a 3-hour collection of *asp*^{*dd1*}-derived embryos in the absence (A) or presence (C) of purified Asp protein (estimated concentration of 5X10⁻⁴ pmol/µl). The inset in C shows an MTOC at higher magnification. B is the same field as pannel C immunostained to reveal γ-tubulin. In this experiment, 70% of γ-tubulin staining bodies were seen to nucleate asters of microtubules. Scale bars, 10 µm. D and E: Purification of Asp. D, silver stained SDS-PAGE gel and E, the corresponding Immunoblot probed with antibodies to Asp and β-tubulin. Lane 1, total extract; lanes 2-4, soluble fractions following sequential washes of GTP-taxol pelleted mictotubules with 500 mM NaCl, 750 mM NaCl and 1M KI.

### EXAMPLES

### Materials and Methods

Immunostaining of *Drosophila* brains was carried out as previously described (Gonzalez *et al*., 1990). DNA was stained with TOTO3 (Molecular Probes) according to the suppliers instructions. Mitotic spindles were visualized by incubation with anti-α-tubulin monoclonal antibody (clone YL1/2 from Harlan Sera Labs) diluted 1:10. We detected γ-tubulin, using the monoclonal antibody from clone GTU88 (Sigma) at a 1:10 dilution. Anti-Asp was policlonal rabbit serum Rb3133 (Saunders *et al*., 1997) diluted 1:50. Secondary antibodies were purchased from Jackson Immunochemicals and used according to the suppliers instructions. Preparations were visualised in a BioRad 1024 confocal scanning head in conjunction with a Nikon Optiphot microscope.

Centrosomes were purified from *Drosophila* embryos according to published protocols for the preparation of centrosomes from chinese hamster ovary cells (Barton *et al*., 1995) The final centrifugation was through a 20-62.5% (w/w) sucrose gradient over a 70% sucrose cushion in a SW27 (Beckman) rotor for 90 min at 65,000 g at 4°C.

Microtubule nucleation assays, extraction with 1 M KI and complementation assays were carried out as previously described (Moritz *et al*., 1998). Microtubules were polymerised from *Drosophila* embryo extracts by addition of guanosine triphosphate (GTP) and taxol (Saunders *et al*., 1997). The microtubule pellet was sequentially extracted with 500 mM NaCl, 750 mM NaCl and 1 M KI. Soluble fractions were concentrated by ultrafiltration using Millipore Ultrafree systems. Tubulin and rhodamin-labelled tubulin used in the centrosome nucleation assays were purchased from Molecular Probes.

Asp was immunodepleted from 100 µl of *Drosophila* embryo extracts by incubation with affinity-purified anti-Asp (20-50 µg) coupled to proteinG-Sepharose beads (Sigma).

SDS-polyacrylamide gel electrophoresis (SDS-PAGE) gels were blotted to polyvinylidene difluoride membranes (Millipore). The following antibodies were used: anti-Asp, Rb3133 (Saunders *et al*., 1997); anti-KLP61F (Barton *et al*., 1995); anti-Polo, MA294; anti-γ-tubulin GTU88 (Sigma); anti-β-tubulin, BX69. All primary antibodies were diluted 1:500, with the exception of MA294 and BX69 which were diluted 1:4. Peroxidase-conjugated secondary antibodies were purchased from Jackson Immunochemicals and used according to the suppliers instructions. Bound antibodies were detected by chemi-luminescence using chemicals from Amersham (ECL) or Pierce (Supersignal).

### Results

Asp is a 220 kDa microtubule-associated protein (MAP) found at the poles of mitotic spindles in the syncytial embryos of *Drosophila melanogaster*. It has consensus phosphorylation sites for p34^{cdc2} and mitogen-activated protein kinases, and putative binding domains for actin and calmodulin. Mutations in *asp* result in abnormal spindle morphology leading to mitotic arrest, or to a high frequency of meiotic non-disjunction. Because the mitotic defects of *asp* mutants are best studied in the larval central nervous system, we sought to examine its distribution more carefully in cells of whole-mount preparations of this tissue. We found that Asp became and remained associated with the centrosome throughout mitosis (Fig.1, A through C). At telophase, it migrated to microtubules on the spindle side of both daughter nucleus and was not associated with the centrosome in interphase cells. The immunolocalisation of Asp on the telophase spindle in cells of the larval brain is not shown. Asp is found in the region between the nuclei and the central spindle. At all mitotic stages from prophase to anaphase, the Asp protein was asymmetrically localised around the γ-tubulin in the PCM, where it appeared to form a hemi-spherical cup contacting the spindle microtubules (Fig. 1B).

In several *asp* mutations, Asp did not immunostain at the spindle poles. The mutant alleles that we examined are *asp*^{*1*}*, asp*^{*dd1*}*, asp*^{*dd*}⁴,*asp*^{*dd*}⁷ and *asp*^{*dd8*} (White-Cooper *et al*., 1996). The majority of mitotically arrested *asp* cells had bipolar spindles with broad unfocused poles of micotubules (Fig. 2, A and B). In a small proportion of cells, one pole could be sufficiently disorganised so that the spindle appeared monopolar. The γ-tubulin was not present within a well-organized centrosome in these cells but was found in dispersed clumps at the spindle poles (Fig. 2, C and D). This suggested that Asp might be required to maintain the structure of the centrosomal microtubule-organizing center (MTOC) during mitosis.

To confirm that Asp was a centrosomal protein, centrosomes were partially purified from syncytial *Drosophila* embryos undergoing their rapid nuclear division cycles). Imunoblotting experiments indicated that this preparation was enriched in both Asp and γ-tubulin (Fig. 1G). Moreover, these two proteins colocalized by imunofluorescence at *in vitro* organising centres for rhodamine-labeled microtubules (Fig. 1, D through F). In contrast to our observations *in vivo,* Asp was found in all of the *in vitro* MTOCs and was distributed symmetrically. First, this suggests that the extract was in a mitotic-like state, probably due to the dominant effect of the active mitotic protin kinase p34^{cdc2}. Second, it implies that the asymmetric localisation seen in intact cells requires that microtubules make contact with chromosomes to form a spindle. If Asp localises to the "outside" of the centrosome, we wondered whether antibodies to Asp might interfere sterically with microtubule nucleation in the *in vitro* assay. The centrosome preparation was therefore incubated with either affinity-purified anti-Asp or control rabbit immunoglobulins before addition of rhodamine-labeled tubulin. A number of control antibodies have been used, including one against the centrosomal component CP190. None of these would prevent microtubule nucleation by the centrosome preparation. The number of asters of microtubules formed decreased in proportion to the concentration of antibody (Fig.1H). However, the number of γ-tubulin positive bodies remained constant, suggesting that the antibody was blocking microtubule nucleation rather than disrupting the centrosomes.

Extraction of centrosomes with KI effectively destroyed their microtubule-nucleating activity leaving a centrosomal scaffold (Fig. 3C) (Schnakenberg *et al*., 1998; Moritz *et al*., 1998). Ability to nucleate microtubules can be restored to such KI-extracted centrosomes by incubation with the soluble fraction of a *Drosophila* embryonic extract (Fig. 3, D and E) (Moritz *et al*., 1998). Although γ-tubulin and its ring-complex (γTuRC) are required to rescue the aster-forming ability of KI-extracted centrosomes, they are not sufficient and have to be supplemented by a high molecular weight microtubule-associated factor postulated to be perientric (Moritz *et al*., 1998). To test whether Asp protein might be required to reconstitute MTOCs from KI-extracted centrosomes, we immunodepleted Asp from a soluble embryonic extract under conditions where several other centrosomally associated proteins, including γ-tubulin, CP190, KLP61F and Polo, were not removed (Fig. 3G). This immunodepleted extract was unable to restore the ability of KI-extracted centrosomes to nucleate microtubules into asters. However, many linear arrays of microtubules were seen (Fig. 3F), suggesting that the Asp-depleted extract provided microtubule nucleation ability, but that it was not organised into discrete centres.

In contrast to the soluble extract of wild-type embryos, the equivalent soluble fraction prepared from *asp*-derived embryos was unable to restore the ability of KI-extracted centrosomes to nucleate asters of microtubules (Fig. 4A). We then investigated whether addition of purified Asp protein could restore this ability to the mutant embryo extract. Asp co-purifies with microtubules from *Drosophila* embryos but is not released by concentrations of NaCl known to remove most MAPs (Saunders *et al*., 1997). Thus, it seemed that if KI extracts Asp from the centrosomes, it probably would do so from such purified microtubules. We extracted NaCl-washed microtubule preparations with 2 M KI, and a 220 kDa protein recognised by antibodies to Asp was found to be the main component of the resulting supernatant fraction (Fig. 4 D and E). This purified Asp fraction was added to the soluble extract prepared from *asp*-derived embryos and found to restore the ability of KI-extracted centrosomes to nucleate asters of microtubules (Fig. 4, B and C).

Thus, it appears that both Asp and γTuRC are required to restore microtubule nucleating activity to centrosome scaffolds. Because Asp neither co-purifies with the γTuRC nor co-immunoprecipitates with γ-tubulin, it is unlikely to have a direct role in the nucleation process. Rather, the consequences of loss of Asp function upon spindle poles in vivo and upon MTOCs *in vitro* suggest that it is required to organise the γTuRC within the PCM to form a nucleating centre for microtubules at mitosis. In *asp* mutants, a spindle can still form, most likely reflecting the known ability of mitotic chromatin and motor proteins to organize a bipolar spindle in the absence of centrosomes. However, one consequence of the disorganized centrosomes and spindle poles is that the cells arrest in a metaphase-like state, suggesting that the spindle integrity checkpoint has been activated. Asp protein function is likely to be modified later in the mitotic cycle, since it was observed to associate with the microtubules of the telophase spindle, a property consistent with its purification as a MAP. However, the lack of any obvious association with microtubules during interphase suggests that its properties have to be modulated, possibly by phosphorylation, during entry into mitosis in order to activate its essential role in maintaining the coherence of the centrosome at the spindle poles.

### References

C. Gonzalez, R. D. Saunders, J. Casal, 1. Molina, M. Carmena, P. Ripoll and D. M. Glover, J. Cell Sci. **96**, 605 (1990).
J. Casal, C. Gonzalez, F. Wandosell, J. Avila and P. Ripoll, Development **108**, 251 (1990).
B. J. Schnackenberg, A. Khodjakov, C. L. Rieder and R. E. Palazzo, Proc. Nail. Acad. Sci. USA **95,** 9295 (1998).
M. Moritz, Y. Zheng, B. Alberts and K. Oegema, J. Cell Biol. **142,** 775 (1998).
H. White-Cooper, M. Carmena, C. Gonzalez and D. M. Glover, Genetics **144**, 1097 (1996).
N. R. Barton, A. J. Pereira and L. S. Goldstein, Mol. Biol. Cell **6,** 1563 (1995).
A. A. Tavares, D. M. Glover and C. E. Sunkel, EMBO J. **15,** 4873 (1996).
Lupas et al., Science **252,** 1162-1164 (1991).
Cheney and Mooseker, Current Opinion in Cell Biology **41**, 27-35 (1992).

### SEQUENCE LISTING

SEQ I.D. No. 1 - Drosophila Asp amino acid sequence (Genbank Accesion No. U95171).

1 MELVWSPVLE VACKETLQLI DNRNFRKEVM IILKSKSNQP VKNPRKFPTV
GKTLQLKSPT
61 GAGKTMKSVV SAAVQQKKRM SAAAAPPSKQ TWRVTAPSRP AAWAHPPPQA
PLVEKNVYKT
121 PQEEPVYISP QPRSLKENLS PMTPGNLLDV IDNLRFTPLT ETRGKGQATI
FPDNLAAWPT
181 PTLKGNVKSC ANDMRPRRIT PDDLEDQPAT NKTFDVKHSE TINISLDTLD
CSRIDGQPHT
241 PLNKTTTIVH ATHTRALACI HEEEGPSPPR TPTKSAIHDL KRDIKLVGSP
LRKYSESMKD
301 LSLLSPQTKY AIQGSMPNLN EMKIRSIEQN RYYQEQQIQI KAKDLNSSSS
SEASLAGQQE
361 FLFNHSEILA QSSRFNLHEV GRKSVKGSPV KNPHKRRSHE LSFSDAPSNE
SLYRNETVAI
421 SPPKKQRVED TTLPRSAAPA NASARSSSAH AWPHAQSKKF KLAQTMSLMK
KPATPRKVRD
481 TSIQPSVKLY DSELYMQTCI NPDPFAATTT IDPFLASTMY LDEQAVDRHQ
ADFKKWLNAL
541 VSIPADLDAD LNNKIDVGKL FNEVRNKELV VAPTKEEQSM NYLTKYRLET
LRKAAVELFF
601 SEQMRLPCSK VAVYVNKQAL RIRSDRNLHL DVVMQRTILE LLLCFNPLWL
RLGLEVVFGE
661 KIQMQSNRDI VGLSTFILNR LFRNKCEEQR YSKAYTLTEE YAETIKKHSL
QKILFLLPFL
721 DQAKQKRIVK HNPCLFVKKS PHKETKDILL RFSSELLANI GDITRELRRL
GYVLQHRQTF
781 LDEFDYAFNN LAVDLRDGVR LTRVVEVILL RDDLTRQLRV PAISRLQRIF
NVKLALGALG
841 EANFQLGGDI AAQDIVDGHR EKTLSLLWQL IYKFRSPKFH AAATVLQKWW
RRHWLHVVIQ
901 RRIRHKELMR RHRAATVIQA VFRGHQMRKY VKLFKTERTQ AAIILQKFTR
RYLAQKQLYQ
961 SYHSIITIQR WWRAQQLGRQ HRQRFVELRE AAIFLQRIWR RRLFAKKLLA
AAETARLQRS
1021 QKQQAAASYI QMQWRTYQLG RIQRHEFLRQ RDLIMFVQRR MRSKWSMLEQ
RKEFQQLKRA
1081 AINIQQRWRA KLSMRKCNAD YLALRSSVLK VQAYRKATIQ MRIDRNHYYS
LRKNVICLQQ
1141 RLRAIMKMRE QRENYLRLRN ASILVQKRYR MRQQMIQDRN AYLRTRKCII
NVQRRWRATL
1201 QMRRERKNYL HLQTTTKRIQ IKFRAKREMK KQRAEFLQLK KVTLVVQKRR
RALLQMRKER
1261 QEYLHLREVT IKLQRRFHAQ KSMRFMRAKY RGTQAAVSCL QMHWRNHLLR
KRERNSFLQL
1321 RQAAITLQRR YRARLNMIKQ LKSYAQLKQA AITIQTRYRA KKAMQKQVVL
YQKQREAIIK

1381 VQRRYRGNLE MRKQIEVYQK QRQAVIRLQK WWRSIRDMRL CKAGYRRIRL
SSLSIQRKWR
1441 ATVQARRQRE IFLSTIRKVR LMQAFIRATL LMRQQRREFE MKRRAAVVIQ
RRFRARCAML
1501 KARQDYQLIQ SSVILVQRKF RANRSMKQAR QEFVQLRTIA VHLQQKFRGK
RLMIEQRNCF
1561 QLLRCSMPGF QARARGFMAR KRFQALMTPE MMDLIRQKRA AKVIQRYWRG
YLIRRRQKHQ
1621 GLLDIRKRIA QLRQEAKAVN SVRCKVQEAV RFLRGRFIAS DALAVLSQLD
RLSRTVPHLL
1681 MWCSEFMSTF CYGIMAQAIR SEVDKQLIER CSRIILNLAR YNSTTVNTFQ
EGGLVTIAQM
1741 LLRWCDKDSE IFNTLCTLIW VFAHCPKKRK IIHDYMTNPE AIYMVRETKK
LVARKEKMKQ
1801 NARKPPPMTS GRYKSQKINF TPCSLPSLEP DFGIIRYSPY TFISSVYAFD
TILCKLQIDM
1861 F

SEQ I.D. No. 2 - Drosophila Asp nucleotide sequence (Genbank Accesion No. U95171).

1 atggagctag tgtggagccc cgtccttgag gtggcctgca aggagacgct
gcagctaata
61 gacaaccgca acttccgaaa ggaggtgatg atcatactca agtccaagag
caaccagccg
121 gtcaagaacc cgcgcaaatt tcctactgtc ggcaagaccc tgcagctgaa
atcgccgaca
181 ggagctggca agacaatgaa aagcgtggta tccgctgctg tgcagcaaaa
gaagcgcatg
241 tctgcagcag cagcgcctcc ctccaagcag acatggcgag tgactgctcc
ttcgcgtccc
301 gctgcatggg cacatccacc tccacaggct cctcttgtcg agaagaatgt
atacaagact
361 ccacaagaag agcccgtata catatcacca cagcctcgca gtcttaagga
aaatctaagc
421 cctatgacgc caggaaacct actcgacgtg attgacaatc tgcgattcac
acctctcacc
481 gaaacccgtg gcaaaggaca agctaccatt ttcccggaca atctggcagc
ctggcccaca
541 ccaacactta aagggaatgt aaaatcatgt gctaatgata tgcggccgcg
tcgaatcact
601 ccagatgatc tagaagatca gcctgccaca aacaaaacgt tcgatgtaaa
gcattccgag
661 actatcaata tttcgttgga caccttggac tgctccagga tcgatggaca
accgcatacg
721 cccctaaata agacaacaac cattgtgcat gccacgcaca ccagagctct
ggcctgtatt
781 catgaggagg agggaccaag tccacctagg acgcccacga agagcgccat
acacgacctg
841 aagagggaca ttaagttggt gggttcaccc ttacgaaagt attccgagtc
catgaaagat

901 ttgtcacttc tatcgccaca aactaagtat gccattcaag ggtctatgcc
taatctaaat
961 gaaatgaaaa tccgctcgat cgaacagaat agatattacc aggagcagca
gatccagata
1021 aaggccaaag acttgaatag ctcctctagt agcgaggcta gtttggccgg
ccagcaggag
1081 tttctattca accacagtga gatcctcgct cagtccagtc gttttaatct
ccatgaagta
1141 ggtcggaagt cggtgaaggg aagtccggtg aagaatcctc acaagcgccg
ctctcatgag
1201 ttgagttttt cggatgcacc tagcaacgaa tcattgtacc gcaatgaaac
tgtagccatt
1261 tcccctccta aaaagcaacg ggttgaggac actactctgc ccagaagtgc
agcgccggca
1321 aatgcatctg caagaagcag tagtgcccac gcctggccac acgcccaatc
caagaagttt
1381 aagctagcac aaaccatgtc actgatgaag aagcccgcca caccacgaaa
agtcagggac
1441 actagcattc agccttccgt caagctctat gactcggagc tgtatatgca
gacgtgcatc
1501 aacccggatc catttgcagc aactacgaca attgatccat ttctggcatc
taccatgtat
1561 ttggatgaac aggctgtgga tcgtcatcaa gctgacttta aaaagtggtt
aaatgccctt
1621 gtctccatac ccgctgacct ggacgcagat ttaaataaca aaatagacgt
tggtaagctg
1681 tttaacgagg tgcgcaacaa agagctcgtg gtggctccca ccaaggagga
gcagtctatg
1741 aactacctaa cgaaataccg cctggagacg cttcgtaagg cggctgtgga
gctcttcttc
1801 agtgagcaga tgcgcctgcc atgctccaaa gtggccgtat atgtcaacaa
gcaagctctg
1861 cgcatccgta gcgatcgtaa tcttcaccta gacgtagtta tgcaacgcac
catacttgag
1921 ctgctgcttt gcttcaatcc cctttggctg cgccttggac tggaagtggt
ctttggcgag
1981 aagatccaga tgcagtctaa tcgagacatt gtaggcctca gcacctttat
cctcaatcgc
2041 ttgttccgga ataagtgtga ggagcagagg tacagcaagg catacacact
caccgaagag
2101 tacgcggaga ccattaagaa gcactcattg cagaaaatcc tctttttgct
gcctttcctc
2161 gatcaagcta agcagaagcg catcgtcaag cacaatccct gtttgtttgt
taaaaagtcg
2221 ccacataaag agaccaagga tattctgctg cgcttctcgt cggagctgct
cgccaacatt
2281 ggtgatatta cgcgggaact tcgtcgcctg ggctacgttc tacagcaccg
ccaaacattt
2341 ttggacgagt tcgattatgc cttcaacaac ttggctgtgg acctaagaga
tggcgtgaga
2401 ctaacccgag tcgtggaggt aattttacta cgcgatgatc taacccgcca
gttaagggtg

2461 ccagccatct ctcgccttca gcggatcttc aatgtaaagc tagctctggg
cgcacttggt
2521 gaagccaact tccagctagg cggcgacatc gccgcccaag acatcgttga
cggacatcgt
2581 gagaagacgc tttccctgct ctggcaactt atttacaaat tccgctcgcc
caagtttcat
2641 gcggcggcca cggtgctcca gaaatggtgg cgccgtcact ggctgcacgt
tgttatccag
2701 cgtcgcattc gccacaaaga gcttatgcgt cgccaccggg ccgctactgt
cattcaggcc
2761 gtgttccgtg gccaccagat gagaaagtac gtgaagttgt ttaagacgga
acgcactcag
2821 gccgcaataa ttctgcaaaa gttcacccgt cggtatttgg cccagaagca
gctatatcag
2881 agctatcaca gtattatcac catccagcgc tggtggcgag cccaacaact
gggaaggcag
2941 caccgccagc ggtttgtgga gctccgagag gctgcgatct ttcttcagcg
gatctggcgg
3001 cgacgactct ttgccaaaaa actattggcg gcggcggaaa cagccagact
tcagcgatcg
3061 caaaaacaac aggcagctgc tagttatatt caaatgcaat ggcgaacgta
tcagctgggc
3121 agaattcagc gacacgagtt cctgcgccag agggacctca tcatgtttgt
tcagcgcagg
3181 atgcgaagca agtggagtat gctggagcag cgcaaggagt tccagcaact
aaagcgtgca
3241 gctataaata tccaacaacg ctggcgagcg aagctttcaa tgagaaagtg
caacgctgat
3301 tatttggcac ttcgttccag cgttcttaaa gttcaggctt acaggaaagc
cacaatccag
3361 atgagaatag atcgtaatca ctactattcc ctgcgaaaaa atgttatctg
cctgcaacag
3421 cgactgaggg ccatcatgaa aatgcgcgaa cagagggaaa attatctgag
gctgcgaaat
3481 gcttcaatac tagttcaaaa acgctaccgc atgcgtcaac aaatgatcca
ggatagaaat
3541 gcatatttaa gaacccgcaa atgtatcatc aatgtccaga ggcgctggag
agccactctg
3601 cagatgcgtc gagaaaggaa gaactacctt catctccaaa cgacaaccaa
acgaattcaa
3661 atcaagttcc gtgccaagcg cgaaatgaaa aagcaaagag ccgagtttct
tcagctgaaa
3721 aaggttaccc ttgtagtcca gaaacgtcgg cgggctttgc tgcagatgcg
aaaggagcgc
3781 caggagtacc tacatctgcg cgaggttacc ataaagctgc agcgcagatt
ccatgctcaa
3841 aaatcaatgc ggttcatgcg agccaagtac cgcggcaccc aggctgctgt
gagctgcctg
3901 cagatgcatt ggcgtaatca tctccttagg aaacggagag ggaacagttt
cttacaactg
3961 cgtcaagcag caataacact gcagcgacgg taccgagctc gtctgaatat
gatcaagcag

4021 ttgaagagtt acgcccagct gaaacaggca gctattacca ttcaaacccg
atatagagcc
4081 aagaaggcaa tgcaaaagca ggtggtcttg taccaaaagc aaagggaagc
cattatcaaa
4141 gtgcaacgac gataccgcgg caatctggag atgaggaagc agattgaagt
ctaccaaaaa
4201 cagcgccagg cagtcatccg cttgcagaaa tggtggcgca gtatacgcga
catgcggctg
4261 tgcaaagcgg gctaccgaag gattcgactc agttcattga gcattcaacg
caagtggcgg
4321 gccacagtgc aggctcgtcg ccaacgggag atcttcttaa gcaccatccg
caaagtgcga
4381 cttatgcagg catttatcag agcaacttta ctgatgcgtc agcagcgcag
ggaattcgag
4441 atgaagcgaa gggctgccgt agtgatccag cgccggtttc gtgctcgctg
tgctatgcta
4501 aaggcgagac aggattacca attaatccaa tcctctgtga tcctggtgca
gcgcaaattc
4561 cgtgctaatc gcagcatgaa gcaggcacgc caggaatttg tccagctgcg
tactattgca
4621 gtccatttgc aacaaaagtt ccgtggcaag cgtctaatga ttgagcagcg
taattgtttc
4681 caactgctcc gctgttccat gccgggcttc caggcacgtg cccgcggctt
tatggctcgc
4741 aaacgattcc aggccctgat gacacccgag atgatggacc tcatccgcca
gaagcgcgcc
4801 gccaaggtta tacagcgtta ctggcggggc tatctaatcc gacgacgtca
gaagcaccag
4861 ggtctcttgg atatccggaa gcgtattgct cagctgcggc aagaggcaaa
ggcggtaaac
4921 tctgtgcgct gcaaagtcca ggaggcggtg cgtttccttc gcggacgctt
tatcgcatca
4981 gatgcgttag cagtgctaag tcaattggat cgtctttcgc gcactgtgcc
acacctgctc
5041 atgtggtgtt cggagttcat gtccacgttt tgctatggca tcatggctca
ggccattcga
5101 tcagaggtgg ataagcagct tatagagcgc tgcagccgga tcatcctaaa
tttggcccgc
5161 tacaatagca ccacggtgaa cacgttccag gagggcggtt tggtcaccat
tgcccagatg
5221 ttattgcgct ggtgcgacaa agacagtgag atattcaaca ctttgtgcac
cctcatttgg
5281 gtattcgccc actgtcctaa aaagcgaaag atcattcacg actatatgac
caacccagag
5341 gccatttaca tggtgcgcga aactaagaag ctcgtggccc gcaaggaaaa
gatgaagcaa
5401 aatgcacgca agccgccgcc aatgacaagt ggacggtata agagccaaaa
gataaacttc
5461 acgccgtgtt ccctgcccag cctggagccg gacttcggaa tcatccgcta
cagtccctac
5521 acgtttatct cgtccgttta cgccttcgat acgattttgt gcaagctgca
gatcgacatg
5581 ttttag

## Claims

1. A method for identifying a substance capable of disrupting microtubule organising centre (MTOC) integrity, which method comprises
contacting an Asp polypeptide as shown in SEQ ID NO:1 or fragment thereof capable of forming and/or maintaining MTOCs in the absence of the substance, with a candidate substance in the presence of components required for MTOC formation and microtubule nucleation therefrom, and measuring microtubule nucleation from centrosomes in the presence of the candidate substance compared to in the absence of the candidate substance to determine whether the substance disrupts MTOC integrity.

2. A method according to claim 1 wherein said components comprise KI-extracted centrosomes and an Asp-depleted soluble cellular extract.

3. A method according to claim 1 wherein said components comprise a centrosome preparation and tubulin.

4. A method according to any one of claims 1 to 3 further comprising administering a substance, which has been determined to disrupt MTOC integrity, to a cell and determining whether said substance inhibits mitosis in the cell.

5. Use of an Asp polypeptide as shown in SEQ ID NO:1 or fragment thereof capable of stimulating formation of and/or maintaining MTOCs, in an assay for identifying a substance capable of disrupting MTOC integrity comprising measuring microtubule nucleation from centrosomes in the presence of the candidate substance compared to in the absence of the candidate substance.

## Patentansprüche

1. Verfahren zur Identifizierung einer Substanz, die in der Lage ist, die Integrität des Mikrotubuli organisierenden Zentrums (MTOC) zu stören, wobei das Verfahren umfaßt,
daß man ein Asp-Polypeptid, wie es in SEQ ID NO:1 gezeigt ist, oder ein Fragment davon, welches in der Lage ist, in der Abwesenheit der Substanz MTOCs auszubilden und/oder zu erhalten, mit einer Kandidatensubstanz in der Gegenwart von Bestandteilen, die für MTOC-Bildung und Mikrotubuluskeimbildung daraus erforderlich sind, in Kontakt bringt und die Mikrotubuluskeimbildung aus Zentrosomen in der Gegenwart der Kandidatensubstanz im Vergleich zu der Abwesenheit der Kandidatensubstanz mißt, um zu bestimmen, ob die Substanz die MTOC-Integrität stört.

2. Verfahren nach Anspruch 1, wobei die Bestandteile mit KI extrahierte Zentrosomen und einen von Asp abgereicherten Zellextrakt umfassen.

3. Verfahren nach Anspruch 1, wobei die Bestandteile eine Zentrosomenpräparation und Tubulin umfassen.

4. Verfahren nach einem der Ansprüche 1 bis 3, welches weiterhin umfaßt, daß man eine Substanz, von der festgestellt wurde, daß sie MTOC-Integrität stört, an eine Zelle verabreicht und bestimmt, ob diese Substanz die Mitose in der Zelle hemmt.

5. Verwendung eines Asp-Polypeptids, wie es in SEQ ID NO:1 gezeigt ist, oder eines Fragments davon, welches in der Lage ist, die Bildung von MTOCs zu stimulieren und/oder MTOCs zu erhalten, in einem Test zur Identifizierung einer Substanz, die in der Lage ist, die MTOC-Integrität zu stören, welcher umfaßt, daß man die Mikrotubuluskeimbildung von Zentrosomen in der Gegenwart einer Kandidatensubstanz im Vergleich zur Abwesenheit der Kandidatensubstanz mißt.

## Revendications

1. Méthode pour identifier une substance capable de rompre l'intégrité des centres d'organisation des microtubules (MTOC), méthode qui comprend
la mise en contact d'un polypeptide Asp représenté dans la SEQ ID N° 1 ou un de ses fragments capables de former et/ou de maintenir les MTOC en l'absence de la substance, avec une substance candidate en présence de constituants requis pour la formation de MTOC et la nucléation des microtubules à partir de ceux-ci, et la mesure de la nucléation des microtubules à partir des centrosomes en présence de la substance candidate, comparativement à celle-ci en l'absence de la substance candidate pour déterminer si la substance provoque la rupture de l'intégrité des MTOC.

2. Méthode suivant la revendication 1, dans laquelle lesdits constituants comprennent des centrosomes ayant subi une extraction au KI et un extrait cellulaire soluble présentant une déplétion en Asp.

3. Méthode suivant la revendication 1, dans laquelle lesdits constituants comprennent une préparation de centrosomes et de la tubuline.

4. Méthode suivant l'une quelconque des revendications 1 à 3, comprenant en outre les étapes consistant à administrer une substance, qui a été déterminée en tant que substance provoquant la rupture de l'intégrité des MTOC, à une cellule et à déterminer si ladite substance inhibe la mitose dans la cellule.

5. Utilisation d'un polypeptide Asp représenté dans la SEQ ID N° 1 ou un de ses fragments capables de stimuler la formation des et/ou de maintenir les MTOC, dans une analyse pour identifier une substance capable de provoquer la rupture de l'intégrité des MTOC, comprenant la mesure de la nucléation des microtubules à partir des centrosomes en présence de la substance candidate, comparativement à celle-ci en l'absence de la substance candidate.
